# EUROPEAN PATENT SPECIFICATION

(11) **EP 1 721 884 B1**
(45) Date of publication and mention of the grant of the patent: **13.08.2014**
(21) Application number: 04792209.1
(22) Date of filing: 08.10.2004
(51) Int. Cl.: C07C 51/46

(54) **PROCESS FOR PRODUCING (METH)ACRYLIC ACID**
VERFAHREN ZUR HERSTELLUNG VON (METH)ACRYLSÄURE
PROCÉDÉ DE PRODUCTION DE L'ACIDE (MÉTH)ACRYLIQUE

(30) Priority: 02.03.2004 JP 2004057253
(43) Date of publication of application: 15.11.2006
(73) Proprietor: Mitsubishi Chemical Corporation, Chiyoda-ku Tokyo 100-8251 (JP)
(72) Inventor: YADA, Shuhei, C/O MITSUBISHI CHEMICAL CORPORATION, Minato-ku Tokyo 108-0014 (JP); TAKASAKI, Kenji, C/O MITSUBISHI CHEMICAL CORP., Yokkaichi-shi, Mie 5108530 (JP); SUZUKI, Yoshiro, C/O MITSUBISHI CHEMICAL CORP., Yokkaichi-shi, Mie 5108530 (JP); OGAWA, Yasushi, C/O MITSUBISHI CHEMICAL CORP., Yokkaichi-shi, Mie 5108530 (JP)
(74) Representative: HOFFMANN EITLE
(86) International application number: PCT/JP2004/014940
(87) International publication number: WO 2005/085167

(56) References cited:
- JP-A- 8 040 974
- JP-A- 2000 281 617
- JP-A- 2001 247 510

## Description

### Technical Field

The present invention relates to a method for producing (meth)acrylic acid, and more particularly to a method for producing (meth)acrylic acid for stably purifying through distillation (meth)acrylic acid over a long period of time while preventing polymerization of (meth) acrylic acid in purification of a (meth) acrylic acid solution obtained through a vapor-phase catalytic oxidation reaction of propane, propylene, acrolein, isobutylene, or t-butyl alcohol using a distillation column.

In the present specification, "(meth)acrylic acid" is a general term for acrylic acid and methacrylic acid, and may refer to one or both thereof.

### Technical Field

(Meth)acrylic acid is industrially important as a raw material for super absorbent polymers or as a raw material for various (meth)acrylates. (Meth)acrylic acid has been recently produced through a vapor-phase catalytic oxidation reaction of propane, propylene, isobutylene, or the like. However, (meth)acrylic acid is highly polymerizable and a solid product is often produced through a polymerization reaction during purification of (meth)acrylic acid through distillation, thereby causing troubles such as clogging of devices to possibly hinder a stable continuous operation.

Thus, an example of a known technique for suppressing formation of a solid product in production of (meth)acrylic acid involves adding various polymerization inhibitors (hydroquinone, phenothiazine, copper carbamate, an N-oxyl compound, air, and the like) during distillation (see Eizo Omori, "Acrylic acid and its polymer (I), Shokodo Co., Ltd. , 1973; JP 07-252477 A; JP 07-228548 A; and JP 10-175912 A, for example). A production operation and a production apparatus are devised to reduce a high temperature portion and an accumulation portion in the production apparatus for (meth)acrylic acid as much as possible for suppressing the undesirable polymerization reaction (see JP 08-239341 A, for example).

However, those countermeasures alone are not sufficient for suppressing polymerization in the purification step for (meth)acrylic acid, and an emergence of a polymerization prevention technique at a higher level has been desired for attaining a stable continuous operation.

US 6,448,438 B1 discloses a method for purifying acryl acid while preventing polymerization of acrylic acid, comprising carrying out dehydration distillation of an aqueous solution of acryl acid by means of a dehydration column.

### Disclosure of the Invention

An object of the present invention is to provide a method for suppressing an undesirable polymerization reaction of (meth)acrylic acid in a purification step for producing (meth)acrylic acid and for avoiding the occurrence of troubles due to clogging of devices or the like, to thereby attain a stable continuous operation.

The inventors of the present invention have conducted various studies for solving the above-mentioned problems, and have found out that polymerizability of (meth)acrylic acid increases when water is present within a certain range of concentration. Based on the finding, the inventors of the present invention have found out that the occurrence of troubles of polymerization can be avoided and a stable continuous operation can be attained by starting supply of (meth)acrylic acid after adjusting a water concentration in a column bottom liquid of a distillation column (hereinafter, a distillation column containing an azeotropic solvent for carrying out distillation may also be referred to as "azeotropic distillation column") to a specific concentration or less at the start of an operation of the distillation column, and thus have completed the present invention.

That is, the gist of the present invention is described below.
1. A method for producing (meth)acrylic acid as defined in claim 1.
2. The method for producing (meth)acrylic acid, in which: (1) a (meth)acrylic acid solution obtained by bringing a reaction gas containing (meth)acrylic acid obtained through a vapor-phase catalytic oxidation reaction into contact with an absorption solvent for absorbing (meth)acrylic acid; or (2) a (meth)acrylic acid solution obtained by extracting (meth)acrylic acid from the aqueous solution of (meth)acrylic acid by using an extraction solvent for extracting (meth) acrylic acid from the aqueous solution of (meth)acrylic acid; is employed as the water-containing liquid of (meth)acrylic acid.

### Brief Description bf the Drawings

Fig. 1 is a diagram showing an embodiment of the present invention.
Fig. 2 is a diagram showing another embodiment of the present invention.

### Best Mode for carrying out the Invention

Hereinafter, the present invention will be described in more detail.

The present invention relates to a method for producing (meth)acrylic acid comprising: distilling a water-containing liquid of (meth)acrylic acid comprising (meth)acrylic acid and water by using a distillation column in the presence of an azeotropic solvent boiling together with at least water to remove water from the aqueous solution of (meth) acrylic acid through azeotropy with the azeotropic solvent, in which, after distillation of the azeotropic solvent is carried out in the distillation column, the water-containing liquid of (meth)acrylic acid is distilled in the presence of the azeotropic solvent.

The water-containing liquid of (meth)acrylic acid is not particularly limited so long as the solution comprises (meth)acrylic acid and water. Examples of the water-containing liquid of (meth)acrylic acid include an aqueous solution of (meth)acrylic acid, an aqueous solution of (meth)acrylic acid containing an organic solvent, and an organic solution of (meth) acrylic acid containing water.

The method for producing (meth)acrylic acid of the present invention is not particularly limited so long as the method includes a step of removing water from the water-containing liquid of (meth)acrylic acid through azeotropy with an azeotropic solvent by using a distillation column. An example of the method for producing (meth)acrylic acid includes a method including: an oxidation step for carrying out a vapor-phase catalytic oxidation reaction of propane, propylene, acrolein, isobutylene, or t-butyl alcohol as a starting material; a collecting step for collecting a gas containing (meth) acrylic acid from the oxidation step by bringing the gas into contact with an absorption solvent as the water-containing liquid of (meth)acrylic acid; a step of separating through distillation (meth)acrylic acid and water from the water-containing liquid of (meth)acrylic acid by using an appropriate azeotropic solvent; a step of separating through distillation low boiling point impurities such as acetic acid from (meth)acrylic acid continuously; and a step of separating through distillation high boiling point impurities further.

Further examples of recently employed method for producing (meth) acrylic acid to which the present invention may be applied include: a method for producing (meth)acrylic acid including a step of separating through azeotropic distillation low boiling point impurities such as water and acetic acid, and a solvent from (meth)acrylic acid at once; and a method for producing (meth)acrylic acid including a step of extracting (meth)acrylic acid from water using an extraction solvent such as methyl isobutyl ketone, isopropyl acetate, methyl ethyl ketone, toluene, or cyclohexane and separating through azeotropic distillation the extraction solvent and remaining water in the extracted (meth)acrylic acid.

In the present invention, it is preferable to employ as the water-containing liquid of (meth)acrylic acid: (1) a (meth)acrylic acid solution obtained by bringing a reaction gas containing (meth)acrylic acid which was obtained through a vapor-phase catalytic oxidation reaction into contact with an absorption solvent for absorbing (meth)acrylic acid; or (2) a (meth)acrylic acid solution obtained by extracting (meth) acrylic acid from the aqueous solution of (meth)acrylic acid by using an extraction solvent for extracting (meth)acrylic acid from the aqueous solution of (meth)acrylic acid. The (meth) acrylic acid solution in the item (1) or (2) can be obtained through the above-mentioned method for producing (meth)acrylic acid.

The absorption solvent is not particularly limited so long as the solvent can absorb gaseous (meth) acrylic acid. Water or a condensate of the gas containing (meth)acrylic acid is usually used as the absorption solvent. An organic solvent described above such as methyl isobutyl ketone may also be used as the absorption solvent.

The extraction solvent is not particularly limited so long as the solvent can extract (meth)acrylic acid from the aqueous solution of (meth)acrylic acid. The organic solvent exemplified above can be used as the extraction solvent.

The azeotropic solvent is selected from solvents each forming an azeotropic mixture with water or with water and acetic acid. Examples of a solvent boiling together with water include n-butyl acetate, isobutyl acetate, isopropyl acetate, and methyl isobutyl ketone. Examples of a solvent boiling together with water and acetic acid include toluene, heptane, cyclohexane, and diisobutyl ether.

Various impurities may be present in the water-containing liquid of (meth)acrylic acid obtained through the above-mentioned method for producing (meth)acrylic acid. Examples of low boiling point impurities include formaldehyde, acetaldehyde, propionaldehyde, isobutylaldehyde, acrolein, methacrolein, formic acid, acetic acid, propionic acid, an azeotropic solvent, and water. Examples of high boiling point impurities include propionic acid, crotonic acid, isobutyric acid, benzaldehyde, furfural, benozoic acid, phenol, ß-hydroxypropionic acid, ß-hydroxyisobutyric acid, ß-acryloxypropionic acid, β-methacryloxyisobutyric acid, and a polymerization inhibitor.

Among the numerous impurities, one of the impurities which must have a controlled concentration in the present invention is water. The reason is because the inventors of the present invention have found out as experimental facts that (meth)acrylic acid easily polymerizes and a production of an insoluble polymerized substance increases when a water concentration in (meth)acrylic acid falls within a certain range. A water concentration in (meth)acrylic acid in which the polymerization is accelerated is in a range of about 5 to 20 mass%.

Meanwhile, a water concentration (water concentration with respect to the total amount of (meth)acrylic acid and water) in the water-containing liquid of (meth)acrylic acid produced through a vapor-phase catalytic oxidation reaction is generally 25 to 75 mass% though also depending on a process. Even when an extraction solvent is used, a water concentration in a solution from which (meth) acrylic acid is extracted usually exceeds 10 mass% in industrial extraction of (meth)acrylic acid. Thus, in any method of the above-mentioned methods for producing (meth) acrylic acid, (meth) acrylic acid in the water-containing liquid of (meth)acrylic acid is exposed to an environment at a water concentration in which (meth)acrylic acid easily polymerizes inside an azeotropic distillation column when water is removed through distillation in the azeotropic distillation column.

An operation of a distillation column is usually started by: supplying water to the distillation column, starting distillation of the water which serves as washing with water, and then supplying an actual supply liquid; or supplying an actual supply liquid abruptly for distillation. In those cases, a liquid in a vicinity of a column bottom is maintained at high temperatures and is exposed to an environment at a water concentration of 5 to 20 mass% for a long period of time after the supply of the actual supply liquid until a water concentration in a column bottom liquid reaches several mass% or less. It has become that avoiding polymerization completely is difficult even through countermeasures such as supplying a large amount of a polymerization inhibitor during this time.

Further, the inventors of the present invention have ascertained that an insoluble polymer formed in a column bottom part and a lower part of the column through the non-steady state operation at the start of the operation acts as a trigger for polymer formation during a steady state operation thereafter. The above is presumably ascribable to the following phenomenon. That is, the insoluble polymer formed in a column bottom part or inside the distillation column at the start of the operation causes accumulation of a liquid or inhibition of uniform dispersion of a polymerization inhibitor, to thereby cause even slight polymerization of (meth)acrylic acid in a steady state operation. Even in a steady state operation, the insoluble polymer is continuously formed in a liquid in the distillation column as described above. The formed insoluble polymer causes partial clogging of pores of trays or packing, and a peeled insoluble polymer causes inhibition of flow in drawing tubes, clogging or damaging of a pump, and the like. Based on such verification, the inventors of the present invention have attained the method of the present invention.

That is, the method of the present invention adjusts a residence time to minimum and a temperature to a low temperature as possible in a part in which a water concentration in (meth)acrylic acid falls within the range for accelerating polymerization of (meth)acrylic acid.

When water is supplied to a distillation column in which distillation by an azeotropic solvent is carried out, a liquid flow rate and a gas flow rate inside the distillation column are changed due to the water supplied, and a temperature inside the distillation column is also changed. Inside of the distillation column soon reaches a steady state, and thus, a fluctuation in an amount of water in the distillation column does not cause any problem in usual azeotropic distillation.

However, when (meth)acrylic acid is supplied along with water, a condition in which (meth)acrylic acid easily polymerizes (a state of a high water concentration) is formed locally before a composition inside the distillation column reaches a steady state, thereby causing polymerization inside the distillation column.

Distillation of the azeotropic solvent is carried out in order to avoid the above. Azeotropic distillation of the azeotropic solvent and water remained in the distillation column is carried out, and then supply of the water-containing liquid of (meth)acrylic acid is started.

In the present invention, the azeotropic solvent and the water may be supplied in combination during distillation of the azeotropic solvent. Such an operation allows distillation by the azeotropic solvent previously carried out in the same condition as or similar conditions to the azeotropic conditions of the water-containing liquid of (meth)acrylic acid and the azeotropic solvent. The operation is preferable for: suppressing fluctuations in distillation conditions from those of azeotropic distillation of the water remained in the distillation column and the azeotropic solvent to those of the azeotropic distillation of the water-containing liquid of (meth)acrylic acid and the azeotropic solvent; and suppressing formation of a polymerized product.

The method of the present invention will be described below in detail.

At the start of an operation of an azeotropic distillation column, an azeotropic solvent is supplied first. Distillation is carried out until water remained in the distillation column reaches a predetermined concentration or less, and then supply of the water-containing liquid of (meth)acrylic acid is started. The water remained in the distillation column is water remained after distillation of water carried out during shutdown before the start of the operation, or water remained after distillation of water carried out at the start of the operation before the distillation of the azeotropic solvent. The water is present inside the distillation column, although in a small amount, unless a special operation is carried out.

The supply of the azeotropic solvent is stopped after supplying an amount to hold up in the azeotropic distillation column of when a total reflux state is maintained. As operating conditions of the azeotropic distillation column in this case, an operation temperature and an operation pressure in steady state are preferably employed. Further, a supply amount of a polymerization inhibitor is preferably the same as that during a steady state operation. The operating conditions of the azeotropic distillation column may be adjusted by supplying water at an amount corresponding to an amount of water in the water-containing liquid of (meth)acrylic acid along with the azeotropic solvent to the azeotropic distillation column.

An endpoint of the distillation of the azeotropic solvent is not particularly limited so long as an amount of water remained in the azeotropic distillation column can be sufficiently reduced through distillation of the azeotropic solvent such that (meth)acrylic acid is not exposed to the conditions, in which (meth)acrylic acid easily polymerizes, for a long period of time during azeotropy of the water-containing liquid of (meth)acrylic acid and the azeotropic solvent. The endpoint of the distillation of the azeotropic solvent is determined by a water concentration in a column bottom liquid.

In the present invention, distillation of an aqueous solution of (meth)acrylic acid in the presence of an azeotropic solvent is carried out by supplying the water-containing liquid of (meth)acrylic acid to a distillation column when or after a water concentration in a column bottom liquid of the distillation column in which the distillation of the azeotropic solvent is carried out reaches 4 mass% or less, for suppressing formation of a polymerized product.

In the distillation of the azeotropic solvent, a water concentration in the column bottom is illustrated at the time the operation reaches a total reflux state. If the water concentration is 4 mass% or less, supply of the water-containing liquid of (meth)acrylic acid is started. In this case, a water concentration in the column bottom is more preferably 1 mass% or less. If a water concentration in the column bottom liquid is more than 4 mass%, a total reflux operation by the azeotropic solvent is further continued, and when a water concentration reaches 4 mass% or less, the supply of the water-containing liquid of (meth)acrylic acid is started.

As the azeotropic distillation column, a known distillation column such as a plate column or a packed column may be employed. The number of theoretical plates of the azeotropic distillation column is preferably 3 or more, and may be selected appropriately from the viewpoints of distillation capacity, equipment cost, and the like. In general, the number thereof is preferably about 5 to 25. Known trays may be used as trays provided in the plate column regardless of the presence or absence of a downcomer. Examples of the trays include bubble cap trays, perforated-plate trays, bubble trays, SUPERFRAC trays, MAX-FRAC trays, and dual flow trays.

The azeotropic distillation column may be packed with various types of packing. The packing may be structured or random in shape. Examples of structured packing include: SULZER PACKING available from Sulzer Brothers Ltd.; SUMITOMO-SULZER PACKING available from Sumitomo Heavy Industries, Ltd.; MELLAPAK available from Sumitomo Heavy Industries, Ltd.; GEM-PAK available from Koch-Glitsch, LP; MONTZ-PAK available from Julius Montz GmbH; GOOD ROLL PACKING available from Tokyo Tokushu Kanaami K. K. ; HONEYCOMB PACK available fromNGK Insulators, Ltd.; IMPULSE PACKING available from Nagaoka International Corporation; and MC PACK available from Mitsubishi Chemical Engineering Corporation. Examples of random packing include: INTALOX SADDLES available from Saint-Gobain NorPro; TELLERETT available from Nittetsu Chemical Engineering Ltd.; PALL RINGS available from BASF Aktiengesellschaft; CASCADE MINI-RING available from Mass Transfer Ltd.; and FLEXI RINGS available from JGC Corporation.

One or more types of the trays or packing may be used in the azeotropic distillation column. In the present invention, the trays and packing may be used in combination.

The distillation of the water-containing liquid of (meth)acrylic acid in the presence of an azeotropic solvent is preferably carried out under the conditions in which a water concentration in the column bottom liquid is not increased to accelerate polymerization, for suppressing formation of a polymerized product. The conditions can be adjusted arbitrarily according to various conditions such as the type of the azeotropic solvent, a water content of the water-containing liquid of (meth)acrylic acid, a boiling point of an azeotropic substance, a pressure in the distillation column, and a supply amount of each of the water-containing liquid of (meth)acrylic acid and the azeotropic solvent to the distillation column.

Examples of the conditions include a column top temperature of 30 to 60°C and a pressure inside the distillation column of 10 to 30 kPa when an aqueous solution of (meth) acrylic acid prepared by absorbing a reaction gas obtained through a vapor-phase catalytic oxidation reaction in water is used as the water-containing liquid of (meth)acrylic acid and when toluene is used as the azeotropic solvent.

In the present invention, another known technique for suppressing polymerization of (meth)acrylic acid may be employed in combination without ruining the effect of the present invention. When the present invention is combined with a conventional polymerization prevention technique of (meth)acrylic acid, an undesirable polymerization reaction in a purification step of (meth)acrylic acid may be suppressed more effectively.

A technique to be combined is not particularly limited to any conventional method. However, a typical method involves addition of a polymerization inhibitor along with a molecular oxygen-containing gas, in particular, air to a distillation column. Examples of the polymerization inhibitor include: phenol-based polymerization inhibitors such as hydroquinone; amine-based polymerization inhibitors such as phenothiazine, phenylenediamines, and N-oxyl compounds; and metal salt-based polymerization inhibitors such as salt of copper dialkyl dithiocarbamate and salt of copper acrylate.

Minimizing factors causing an undesirable polymerization reaction of (meth)acrylic acid such as a high temperature portion, a long residence time portion, an accumulation portion (dead space), and protrusions in the azeotropic distillation column is preferable for further exerting the effect of the present invention.

In the present invention, the azeotropic solvent or the extraction solvent may be removed through distillation from the water-containing liquid of (meth)acrylic acid supplied to the azeotropic distillation column after or while removing water in the water-containing liquid of (meth)acrylic acid through distillation of the azeotropic solvent. The removed azeotropic solvent or extraction solvent may be recycled in the next production of (meth)acrylic acid.

Hereinafter, embodiments of the present invention will be described in more detail.

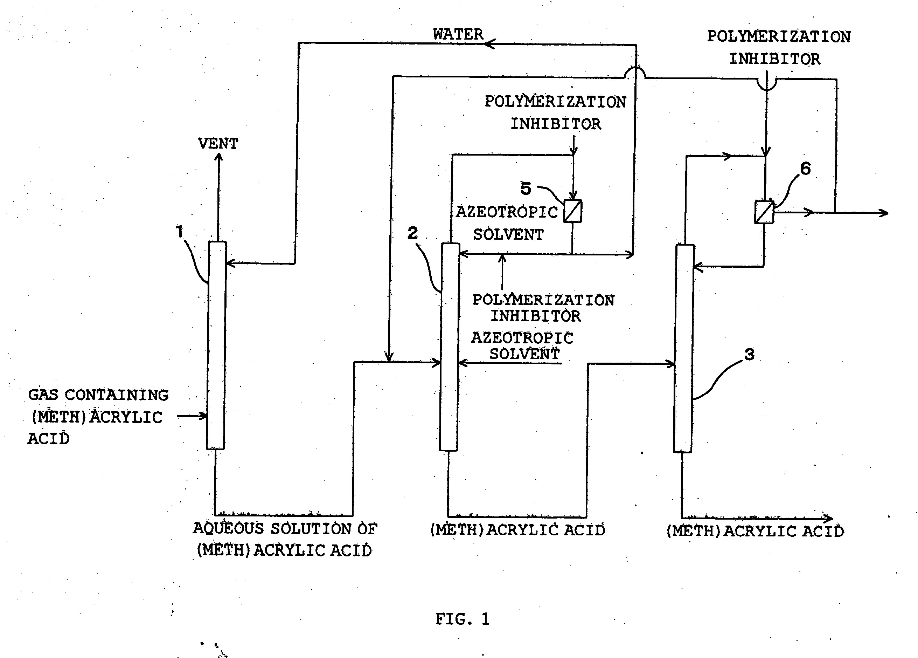

A preferable embodiment of the present invention includes a production method shown in Fig. 1. The production method employs a production apparatus provided with: a collection column 1 for collecting a gas containing (meth)acrylic acid obtained through a vapor-phase catalytic oxidation reaction in water as an absorption solvent; an azeotropic distillation column 2 for distilling water in an aqueous solution of (meth)acrylic acid by boiling the aqueous solution of (meth)acrylic acid obtained in the collection column 1 together with an azeotropic solvent; and a low boiling point substance separation column 3 for separating a low boiling point substance (acetic acid or the like) from (meth)acrylic acid from which water is removed in the azeotropic distillation column. The azeotropic distillation column 2 and the low boiling point substance separation column 3 are each provided with a condenser (not shown) for condensing a vapor from a column top and a receiver 5 or 6 for receiving the obtained condensate.

In the collection column 1, (meth) acrylic acid in the gas containing (meth)acrylic acid is collected in water through a gas liquid contact of the gas containing (meth)acrylic acid and water. A gas component in the gas containing (meth)acrylic acid, which is not collected in water, is discharged as a vent gas.

In the azeotropic distillation column 2, distillation of an azeotropic solvent is carried out before azeotropic distillation of the aqueous solution of (meth)acrylic acid. A vapor containing water remained in the azeotropic distillation column 2 and the azeotropic solvent is condensed in the condenser and received in the receiver 5. A polymerization inhibitor is supplied to the condenser to suppress formation of a polymerized product in the condenser or the receiver 5.

A condensate received in the receiver 5 is separated into water and the azeotropic solvent. The water is returned to the collection column 1 and used for collecting (meth)acrylic acid. A polymerization inhibitor for suppressing occurrence of polymerization in the azeotropic distillation column 2 is supplied to the azeotropic solvent, and the azeotropic solvent is returned to the azeotropic distillation column 2.

When a water concentration in a column bottom liquid of the azeotropic distillation column 2 is reduced to an appropriate value through such operations, the aqueous solution of (meth)acrylic acid is supplied to the azeotropic distillation column 2 to remove water from the aqueous solution of (meth) acrylic acid through azeotropic distillation.

In the low boiling point substance separation column 3, a (meth)acrylic acid liquid, from which water is removed, obtained from the column bottom liquid of the azeotropic distillation column 2 is purified through distillation. A vapor containing acetic acid, (meth)acrylic acid, and the like from a column top of the low boiling point substance separation column 3 is condensed in the condenser and received in the receiver 6 as a condensate. A polymerization inhibitor is supplied to the condenser, to thereby suppress formation of a polymerized product in the condenser or the receiver 6. The condensate received in the receiver 6 may be partially returned to the low boiling point substance separation column 3, may be partially returned to the azeotropic distillation column 2, or may be partially separated from acetic acid for recovering purified (meth)acrylic acid.

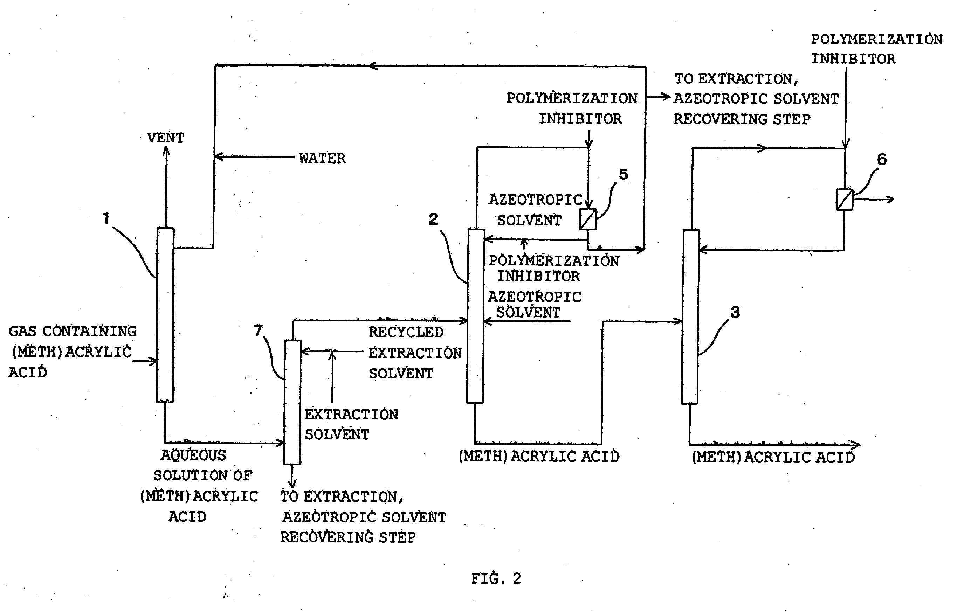

Further, another preferable embodiment of the present invention includes a production method shown in Fig. 2. The production method employs the same production apparatus as that described above except that the apparatus is provided with an extraction column 7 for extracting in an extraction solvent (meth)acrylic acid from the aqueous solution of (meth)acrylic acid obtained in the collection column 1 so that the obtained (meth)acrylic acid extract is supplied to the azeotropic distillation column 2.

The aqueous solution of (meth)acrylic acid obtained in the collection column 1 is received in the extraction column 7. In the extraction column 7, (meth)acrylic acid in the aqueous solution is extracted into the extraction solvent, to thereby provide a (meth)acrylic acid extract. A water phase of the extraction column 7 may be returned to the collection column 1 to be used for collecting (meth)acrylic acid or may be delivered to a step of recovering the extraction solvent from the water phase. The same solvent as the azeotropic solvent is used as the extraction solvent. A novel extraction solvent, or the azeotropic solvent or extraction solvent recovered in the production of (meth)acrylic acid is used as the extraction solvent.

In the azeotropic distillation column 2, the (meth)acrylic acid extract is distilled after the distillation of the azeotropic solvent as described above is carried out, to thereby remove the extraction solvent and water from the (meth)acrylic acid extract. Purified (meth)acrylic acid is obtained in the same manner.

Each of the embodiments described above suppresses an undesirable polymerization reaction of (meth)acrylic acid in a purification step for producing (meth)acrylic acid, avoids troubles due to clogging of devices or the like, and can attain a stable continuous operation.

### Examples

Hereinafter, the present invention will be more specifically described with reference to examples and comparative examples, but the present invention is not limited thereto.

### <Example 1>

A distillation column having a diameter of 1,000 mm provided with a reboiler in a bottom part, a condenser in a column top part, and 30 ripple trays inside the distillation column was used as the azeotropic distillation column. An outlet of the condenser was connected to vacuum equipment. The reboiler was provided in a circulation line for circulating a column bottom liquid by drawing part of a liquid in the column bottom part of the azeotropic distillation column (column bottom liquid) from the column bottom part and for supplying the column bottom liquid to the azeotropic distillation column again. A receiver for receiving a condensate formed in the condenser was provided between the condenser and the vacuum equipment. Tubes and the like were arbitrarily connected to the receiver to reflux each of a top layer and a lower layer of the received condensate independently to the azeotropic distillation column, or to draw each of the top layer and the lower layer of the received condensate independently.

Toluene was supplied to the column bottom while a vapor pressure in a column top part was controlled at 15 kPa. Steam (at a flow rate of steady state) was passed through the reboiler so that toluene was in a total reflux state. Toluene was additionally supplied such that a liquid surface of the column bottom or a liquid surface of the receiver at the column top was 50% during total reflux.

Phenothiazine and hydroquinone as polymerization inhibitors were fed from the column top part at a flow rate (each concentration is 500, 800 mass ppm in the column bottom liquid) of a steady state. Air was fed from the column bottom part at 500 L/hr. The water phase accumulated in the receiver was discharged out of the system. Adjusting to a total reflux state required only 6 hours after supply of toluene. The column bottom liquid was sampled at this time and measured, resulting in a water content of 0.75 mass%.

Thus, an acrylic acid solution was supplied to the 16th tray in the azeotropic distillation column under the conditions of a steady state, that is, at 1,000 kg/hr. An average composition of the acrylic acid solution was 60% acrylic acid and 4% acetic acid in mass concentration, and most of the remaining was water. A reflux liquid from the receiver in a steady state operation was refluxed to the 30th tray in the azeotropic distillation column at 3,100 kg/hr for an operation of the azeotropic distillation column.

A continuous distillation operation was carried out at a column top temperature of 44° C for 3 months under such operating conditions. However, no increase in a differential pressure (difference between the column top pressure and the column bottom pressure) in the distillation column was observed.

### <Comparative Example 1>

Azeotropic distillation was started in the same apparatus and under the same conditions as those in Example 1 after supplying toluene and the acrylic acid solution having the same average composition as that in Example 1 without the total reflux operation by toluene before starting the supply of the acrylic acid solution. 24 hours were required after the supply of the acrylic acid solution until a concentration of water in the column bottom became 4 mass% or less.

Then, a continuous operation was carried out for 3 months as in Example 1. As a result, a differential pressure in the distillation column gradually increased from the start of the operation and an increase of 0.9 kPa was observed after 3 months.

### Industrial Applicability

The present invention has a large effect for suppressing an undesirable polymerization reaction of (meth)acrylic acid in the step of removing water from a water-containing liquid of (meth)acrylic acid for producing (meth)acrylic acid, for avoiding troubles due to clogging of devices or the like, and for stabilizing an operation, to thereby attain a stable continuous operation. The use of the present invention suppresses undesirable polymerization of (meth)acrylic acid in the purification step, in particular, in the azeotropic distillation column to enable a stable operation over a long period of time, and thus has an extremely high industrial value.

## Claims

1. A method for producing (meth)acrylic acid comprising:
distilling a water-containing liquid of (meth)acrylic acid comprising (meth)acrylic acid and water by using a distillation column in the presence of an azeotropic solvent, forming an azeotropic mixture with water or with water and acetic acid, to remove water from the water-containing liquid of (meth)acrylic acid through azeotropy with the azeotropic solvent, wherein
after distillation of the azeotropic solvent is carried out in the distillation column, the water-containing liquid of (meth) acrylic acid is distilled in the presence of the azeotropic solvent by supplying the water-containing liquid of (meth)acrylic acid to the distillation column when or after a water concentration in a column bottom liquid of the distillation column, in which the distillation of the azeotropic solvent is carried out, reaches 4 mass% or less, wherein
the water in the column bottom liquid of the distillation column is water remained after distillation of water carried out during shutdown of the distillation column before a start of operation of the distillation column, or water remained after distillation of water carried out at the start of the operation of the distillation column before the distillation of the azeotropic solvent.

2. The method for producing (meth)acrylic acid according to claim 1, wherein: (1) a (meth)acrylic acid solution obtained by bringing a reaction gas containing (meth) acrylic acid which was obtained through a vapor-phase catalytic oxidation reaction into contact with an absorption solvent for absorbing (meth) acrylic acid; or (2) a (meth)acrylic acid solution obtained by extracting (meth)acrylic acid from the aqueous solution of (meth)acrylic acid by using an extraction solvent for extracting (meth) acrylic acid from the aqueous solution of (meth)acrylic acid; is employed as the water-containing liquid of (meth)acrylic acid.

## Patentansprüche

1. Verfahren zur Herstellung von (Meth)acrylsäure, welches umfasst:
Destillation einer wasserhaltigen Flüssigkeit von (Meth)acrylsäure, die (Meth)acrylsäure und Wasser umfasst, unter Verwendung einer Destillationskolonne in der Gegenwart eines azeotropen Lösungsmittels, wodurch eine azeotrope Mischung mit Wasser oder mit Wasser und Essigsäure gebildet wird, um Wasser aus der wasserhaltigen Flüssigkeit von (Meth)acrylsäure durch Azeotropie mit dem azeotropen Lösungsmittel zu entfernen, wobei
nachdem die Destillation des azeotropen Lösungsmittels in der Destillationskolonne durchgeführt wurde, die wasserhaltige Flüssigkeit der (Meth)acrylsäure in der Gegenwart des azeotropen Lösungsmittels durch Zuführen der wasserhaltigen Flüssigkeit der (Meth)acrylsäure in die Destillationskolonne, wenn oder nachdem die Wasserkonzentration in der Kolonnenbodenflüssigkeit der Destillationskolonne, in der die Destillation des azeotropen Lösungsmittels durchgeführt wird, 4 Massen% oder weniger erreicht, destilliert wird, wobei
das Wasser in der Kolonnenbodenflüssigkeit der Destillationskolonne Wasser, welches, nachdem die Wasserdestillation durchgeführt wurde, während des Herunterfahrens der Destillationskolonne vor Inbetriebnahme der Destillationskolonne übrig blieb, oder Wasser, welches, nachdem die Wasserdestillation durchgeführt wurde, bei Inbetriebnahme der Destillationskolonne vor der Destillation des azeotropen Lösungsmittels übrig blieb, ist.

2. Verfahren zur Herstellung von (Meth)acrylsäure gemäß Anspruch 1, wobei: (1) eine (Meth)acrylsäurelösung, die durch Inkontaktbringen eines Reaktionsgases, das (Meth)acrylsäure, die durch eine katalytische Gasphasen-Oxidationsreaktion erhalten wurde, enthält, mit einem Absorptionslösungsmittel zur Absorption von (Meth)acrylsäure erhalten wird; oder (2) eine (Meth)acrylsäurelösung, die durch Extraktion von (Meth)acrylsäure aus der wässrigen (Meth)acrylsäurelösung unter Verwendung eines Extraktionslösungsmittels für die Extraktion von (Meth)acrylsäure aus der wässrigen (Meth)acrylsäurelösung erhalten wird; als die wasserhaltige Flüssigkeit von (Meth)acrylsäure eingesetzt wird.

## Revendications

1. Un procédé de production de l'acide (méth)acrylique comprenant:
la distillation d'un liquide d'acide (méth)acrylique contenant de l'eau comprenant de l'acide (méth)acrylique et de l'eau en utilisant une colonne de distillation en présence d'un solvant azéotropique, formant un mélange azéotropique avec l'eau ou avec l'eau et l'acide acétique, pour éliminer l'eau du liquide d'acide (méth)acrylique contenant de l'eau par azéotropie avec le solvant azéotropique, dans lequel
après avoir effectué la distillation du solvant azéotropique dans la colonne de distillation, le liquide d'acide (méth)acrylique contenant de l'eau est distillé en présence du solvant azéotropique en approvisionnant le liquide d'acide (méth)acrylique contenant de l'eau à la colonne de distillation lorsque ou après qu'une concentration d'eau dans un liquide au bas de la colonne de la colonne des distillation, dans laquelle la distillation du solvant azéotropique est effectué, atteint 4% en masse ou moins, dans lequel
l'eau dans le liquide au bas de la colonne de la colonne de distillation est de l'eau restée après distillation d'eau effectuée pendant l'arrêt de la colonne de distillation avant un démarrage d'opération de la colonne de distillation, ou de l'eau restée après distillation d'eau effectuée au démarrage de l'opération de la colonne de distillation avant la distillation du solvant azéotropique.

2. Le procédé de production de l'acide (méth)acrylique selon la revendication 1, dans lequel: (1) une solution d'acide (méth)acrylique obtenue en mettant en contact un gaz de réaction contenant l'acide (méth)acrylique qui a été obtenu par une réaction d'oxydation catalytique en phase vapeur avec un solvant d'absorption pour absorber l'acide (méth)acrylique; ou (2) une solution d'acide (méth)acrylique obtenu par extraction de l'acide (méth)acrylique de la solution aqueuse d'acide (méth)acrylique en utilisant un solvant d'extraction pour extraire l'acide (méth)acrylique de la solution aqueuse d'acide (méth)acrylique; est employée en tant que liquide d'acide (méth)acrylique contenant de l'eau.
